Europäisches Patentamt

European Patent Office

Office européen des brevets

(11) Veröffentlichungsnummer: **0 284 872 B1**

(12) **EUROPÄISCHE PATENTSCHRIFT**

(45) Veröffentlichungstag der Patentschrift: **07.08.91**

(21) Anmeldenummer: **88104059.6**

(22) Anmeldetag: **15.03.88**

(51) Int. Cl.⁵: **B01J 27/28**, B01J 27/198, B01J 38/12, C07C 51/377, C07C 57/04

(54) Aktivitätsstabilisierung und Reaktivierung von Heteropolysäurekatalysatoren.

(30) Priorität: **17.03.87 DE 3708627**
 **07.03.88 DE 3807364**
 **07.03.88 DE 3807363**

(43) Veröffentlichungstag der Anmeldung:
 **05.10.88 Patentblatt 88/40**

(45) Bekanntmachung des Hinweises auf die
 Patenterteilung:
 **07.08.91 Patentblatt 91/32**

(84) Benannte Vertragsstaaten:
 **BE ES FR IT**

(56) Entgegenhaltungen:
 EP-A- 0 046 840      EP-A- 0 061 018
 EP-A- 0 071 137      EP-A- 0 165 210
 US-A- 3 474 041      US-A- 4 649 205

(73) Patentinhaber: **Röhm GmbH**
 **Kirschenallee Postfach 4242**
 **W-6100 Darmstadt 1(DE)**

(72) Erfinder: **Langerbeins, Klaus, Dr.**
 **Bahnstrasse 31-33**
 **W-6070 Langen(DE)**
 Erfinder: **Jelitte, Rüdiger, Dr.**
 **Egerländer Strasse 24**
 **W-6101 Rossdorf 1(DE)**
 Erfinder: **Ruppert, Wolfgang, Dr.**
 **Weedring 9**
 **W-6104 Seeheim-Jugenheim(DE)**
 Erfinder: **Emig, Gerhard, Prof. Dr.**
 **Vogelherd 157**
 **W-8520 Erlangen(DE)**
 Erfinder: **Watzenberger, Otto**
 **Bismarkstrasse 21**
 **W-8520 Erlangen(DE)**
 Erfinder: **Haeberle, Thomas-Michael, Dr**
 **Lilienstrasse 19**
 **8502 Zirndorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Verhinderung der Desaktivierung bzw. zur Regenerierung von partiell desaktivierten Heteropolysäure-Katalysatoren, die P, Mo und V als wesentliche Elemente enthalten, bei ihrer Verwendung als Oxidationskatalysatoren, insbesondere bei ihrer Verwendung als Katalysatoren für die Oxidehydrierung von Isobuttersäure oder deren niederen Estern.

Heteropolysäuren, insbesondere Phosphor-Heteropolysäuren des Molybdäns wie Molybdophosphorsäure, $H_3PMo_{12}O_{40}$, oder Molybdovanadophosphorsäuren, wie $H_5PMoV_2O_{40}$ und andere, sind als Katalysatoren bei Gasphasenoxidationen, wie etwa der Synthese ungesättigter Carbonsäuren aus ungesättigten Aldehyden oder bei oxidativen Dehydrierungen in der Gasphase, wie der Herstellung ungesättigter Carbonsäuren aus gesättigten Carbonsäuren, wirksam.

Die Heteropolysäuren und/oder Metallsalze derselben werden vorzugsweise auf einem inerten Material aufgebracht als Katalysator angewendet (US-A-4 146 574, US-A-4 370 490). Solche Katalysatoren halten nicht über längere Zeit ihre nach dem Einfahren erreichten Aktivitäts- und Selektivitätsmaxima, wie dies für technische Prozesse notwendig ist. Sie verlieren nach einigen Tagen oder Wochen ständig an Wirksamkeit.

In der JP-A 81 163 755 ist ein Verfahren zur Wiedergewinnung von den Heteropolysäuren zugehöriger Katalysatorkomponenten durch deren Extraktion mit einem wäßrigen Medium aus partiell desaktivierten Heteropolysäure-Träger-Katalysatorkombinationen beschrieben. Der Extraktion ist ein Kontaktieren der Extraktionslösung in der Wärme mit molekularem Sauerstoff angeschlossen oder beide Maßnahmen - Extrahieren und Einwirkung molekularen Sauerstoffs in der Wärme - werden gleichzeitig durchgeführt. Beispielhaft werden die Katalysatoren bei der Oxidehydrierung von Isobuttersäure zu Methacrylsäure eingesetzt. Ein bei der Verwendung der Heteropolysäure-Katalysatoren aufgetretener Molybdänverlust kann dann bei den Wiedergewinnungsmaßnahmen durch Zusetzen von Molybdänoxid oder von Molybdänoxysäure ausgeglichen werden. Aus den gewonnenen Heteropolysäurelösungen werden mit Trägersubstanzen wieder Heteropolysäure z.B. ($H_5PMo_{10}V_2O_{40}$)-haltige Katalysatoren hergestellt, und diese bei der Oxidehydrierung wiederverwendet.

Die DE-A-36 26 255 beschreibt ein Verfahren zur Regenerierung von bei der Oxidehydrierung von Isobuttersäure oder ihren Estern zu Methacrylsäure oder ihren Estern gebrauchten Heteropolysäure-Katalysatoren, das dadurch gekennzeichnet ist, daß ein aus Phosphormolybdänsäure und/oder ihrer Vanadiumderivate und/oder deren Salzen hergestellter Katalysator zur Regenerierung im Temperaturbereich von 200 bis 400 Grad C mit einem sauerstoffhaltigen Gas oxidierend behandelt wird.

Diese bekannten Verfahren des Standes der Technik zur Regenerierung von Oxidehydrierungskatalysatoren auf Heteropolysäure-Basis mit Molybdän als wesentlichem Bestandteil, weisen verschiedene, deutliche Nachteile auf. So ist das Verfahren der JP-A: Extrahieren, d.h. Abtrennung vom Katalysatorträger, relativ langwierige Sauerstoffbehandlung, Aufkonzentrieren des Extraktes, Wiederaufbringung von Heteropolysäure auf Katalysatorträger, Füllen des Reaktors mit Katalysator und erneutes Anfahren der Oxidehydrierung, verfahrenstechnisch und zeitlich sehr aufwendig.

Das Verfahren der DE-A-36 26 255 ist dagegen verfahrenstechnisch einfach und ohne großen Zeitaufwand durchführbar. Mit den danach durchzuführenden Maßnahmen wird jedoch die jeweils vorgegebene Katalysatoraktivität und -selektivität nicht mehr ganz erreicht, so daß in technisch und wirtschaftlich notwendigen Betriebszeiten die katalytische Wirksamkeit relativ rasch abfällt.

Die DE-C 24 35 031 beschreibt ein Verfahren zur Herstellung von Acrylnitril durch Ammonoxidation von Propylen an molybdänhaltigen Metalloxid-Oxidationskatalysatoren, wobei der Katalysator bei Aktivitätsverlust unter Bedingungen der Ammonoxidation durch Einbringung von molybdänhaltigem Träger in die Wirbelschicht regeneriert wird.

Die Aufgabe war, die kontinuierlich fortschreitende Desaktivierung von Katalysatoren aus Heteropolysäuren des Molybdäns während der katalytischen Umsetzung wie insbesondere während der Oxidehydrierung von Isobuttersäure oder ihren niederen Estern zu den entsprechenden Methacrylverbindungen zu verhindern bzw. sie wesentlich zu verlangsamen, um so technisch und wirtschaftlich sinnvolle Betriebszeiten der eingesetzten Katalysatoren zu erreichen.

Es wurde gefunden, daß die Reaktivierung bzw. die Aktivitätsstabilisierung der Katalysatoren durch die Maßnahmen:

1. Zuführung von Katalysatorelementen in Form entsprechender Elementverbindungen in das Katalysatorsystem und

2. oxidierender Behandlung der ergänzten Katalysatoren bei 200 bis 400 Grad C,

erreicht wird, wobei diese Maßnahmen an den Katalysatoren im Reaktorsystem ohne deren Ausbau, entweder schon während, d.h. mit der katalytischen Oxidationsreaktion, wie z.B. der oxidativen Dehydrierung von Isobuttersäure, oder während der oxidativen Regenerierung der gebrauchten Katalysatoren gemäß

EP 0 284 872 B1

der DE-A-36 26 255, durchgeführt werden, oder daß man die Maßnahmen zeitlich und räumlich getrennt durchführt.

Es ist überraschend, daß sich die Katalysator-Spezies, welche bei den Oxidationsreakticnen wirksam sind, aus den zusammengebrachten Komponenten, nämlich den Katalysatorfragmenten und den zur Ergänzung eingebrachten Verbindungen, auch bei Temperaturen von 200 bis 400 Grad C in oxidierender Atmosphäre bilden.

Es hat sich gezeigt, daß bei der Oxidehydrierung von Isobuttersäure an Heteropolysäuren des Molybdäns, insbesondere dieses Element aus der Reaktionszone herausgetragen wird. Im wesentlichen dürfte dies für die relativ starke Abnahme der Katalysator-Langzeit-Aktivität verantwortlich sein. Aber auch andere im Katalysator enthaltene Elemente, wie Vanadium oder Phosphor oder weitere Katalysatorzusätze, werden während der Oxidationsreaktion herausgetragen. Erfahrungsgemäß liegen die Verluste an Nicht-Molybdän-Elementen, normalerweise d.h. wenn der Katalysator nicht ein als relativ flüchtig bekanntes Element enthält, deutlich unter den Molybdänverlusten.

Wesentlich für das neue Verfahren ist, daß eine effektive Katalysatorstabilisierung bzw. Katalysatorregenerierung sowohl aus der Reoxidation der zum Heteropolysäurekatalysator-Verband hehörenden Elemente, wie insbesondere Molybdän und Vanadium und gegebenenfalls weiteren Metallionen, als auch aus einer Neubildung des Heteropolysäurekatalysator-Verbandes mit den erfindungsgemäß eingeführten Verbindungen von Katalysatorelementen, wie des Molybdäns, des Phosphors und/oder des Vanadiums, insbesondere solchen des Molybdäns und/oder Phosphors und gegebenenfalls weiterer wesentlicher Heteropolysäurekomponenten besteht, in sauerstoffhaltiger Atmosphäre stattfindet.

Die Erfindung betrifft ein Verfahren zur Aktivitätsstabilisierung und/oder zur Regenerierung von Katalysatoren aus Heteropolysäuren, die Molybdän, Phosphor und Vanadium als wesentliche Elemente, sowie gegebenenfalls weitere metallische Elemente als Kationen enthalten bei ihrer Verwendung als Oxidationskatalysator in Gasphasenreaktionen, das dadurch gekennzeichnet ist, daß dem Katalysator Verbindungen mit Katalysatorelementen zugeführt werden und der Katalysator in sauerstoffhaltiger, oxidierend wirkender Atmosphäre auf 200 bis 400 Grad C erhitzt wird.

Die Erfindung ermöglicht die vorteilhafte Regenerierung gebrauchten Katalysators bei Temperaturen im Bereich von 200 bis 400 Grad C ohne technisch umständliche Maßnahmen ergreifen zu müssen, bei praktisch kontinuierlich bzw. kontinuierlich-periodisch betriebenem katalytischem Verfahren.

Das katalytisch betriebene Verfahren, bei dem die erfindungsgemäßen Maßnahmen angewendet werden, ist vor allem die oxidative Dehydrierung von Isobuttersäure oder ihrer niederen Ester zu Methacrylsäure oder ihrer niederen Ester, die an Heteropolysäurekatalysatoren im Temperaturbereich von 250 bis 400 Grad C durchgeführt wird.

Die Erfindung betrifft danach auch ein Verfahren zur Regenerierung von Heteropolysäurekatalysatoren, die Molybdän, Phosphor und Vanadium als wesentliche Elemente, sowie gegebenenfalls weitere metallische Elemente als Kationen enthalten, für ihre Verwendung als Oxidationskatalysatoren in Gasphasenreaktionen, insbesondere für die Oxidehydrierung von Isobuttersäure oder ihren niederen Estern zu Methacrylsäure oder ihren niederen Estern, wobei dem aus Phosphormolybdänsäure und/oder ihrer Vanadiumderivate und/oder deren Salzen hergestellten Katalysator zur Regenerierung Verbindungen mit Katalysatorbestandteilen zur Ergänzung der Katalysatorelementen-Verluste zugeführt werden und dieser dann in sauerstoffhaltiger, oxidierend wirkender Atmosphäre auf 200 bis 400 Grad C erhitzt wird.


Ausführung der Erfindung


Die erfindungsgemäße Stabilisierung und/oder Reaktivierung bzw. Regenerierung des Katalysatorsystems findet bei der Oxidationsreaktion oder intermittierend mit dieser statt. In der DE-A- 36 26 255 ist die oxidative Regenerierung von Heteropolysäure-Katalysatoren bei der Oxidehydrierung von Isobuttersäure oder deren Estern bzw. intermittierend mit dieser, ausführlich beschrieben. Das neue Merkmal -Zugeben von Verbindungen mit Katalysatorbestandteilen, insbesondere einer Molybdänverbindung und/oder einer Phosphorverbindung läßt sich mit bekannten Verbindungen und nach bekannten Methoden durchführen.

Als Molybdänverbindungen kommen hierbei solcher der verschiedensten Oxidationsstufen des Molybdäns, wie beispielsweise 2-, 3-, 4-, 6- oder auch gemischtwertige Verbindungen in Frage. Genannt seien organische Verbindungen des Molybdäns, wie z.B. Molybdän-II-actetat, Molybdänoxiacetylate, Molybdän-II-i-butyrat oder Oxiisobutyrate des Molybdäns, weitere Molybdänacylate bzw. Molybdanoxiacylate, Molybdänalkoholate bzw. Oxialkoholate des Molybdäns, oder anorganische Molybdänverbindungen, wie z.B. $MoO_3$, $Mo(CO)_6$, gegebenenfalls Molybdänoxyhalogenide oder gemischtwertige Oxide, wie das Molybdänblau. Die Verbindungen können in vorzugsweise feinteiliger Form während der Regenerierungsphasen mit Hilfe mechanischer Fördermethoden in den katalysatorhaltigen Reaktor eingebracht werden oder sie können in

3

EP 0 284 872 B1

diesen destilliert bzw. sublimiert werden. Beispielsweise entstehen aus metallischem Molybdän beim Erhitzen auf Rotglut in Gegenwart von sauerstoffhaltigen Gasen, z.B. Luft, sublimierbare blaue Molybdänoxide. Unter den bei der Regenerierung herrschenden Bedingungen ≈ 200 bis 400 Grad C, Oxidationsbedingungen und wie weiter schon in der deutschen DE-A-36 26 255 beschrieben, vorteilhaft die Gegenwart von Wasserdampf - werden die eingebrachten Molybdänverbindungen durch hydrolytisch-oxidative Reaktionen in für die Regenerierung der Heteropolysäure-Katalysatoren besonders brauchbare Molybdän-VI-Sauerstoff-Verbindungen überführt.

Ein bevorzugtes Verfahren, Molybdän als gasförmige Spezies in brauchbarer Form zu erzeugen besteht darin, Isobuttersäure durch ein Bett mit einer oxidischen Molybdänverbindung, beispielsweise MoO₃, bei Temperaturen von 150 bis etwa 350 Grad C zu leiten. Die Menge der mitgeführten Molybdänverbindungen, wie z.B. Oxiisobutyrate des Molybdäns, läßt sich mit der Temperatur des Bettes steuern. Der so erhaltene Molybdän-haltige Dampf wird am Eingang zum Heteropolysäurekatalysator-haltiger Reaktor gegebenenfalls mit weiterem umzusetzenden Substrat (Isobuttersäure) und sauerstoffhaltigem Gas vermischt und so zur katalytischen Reaktion gebracht.

Eine vorteilhafte Ausgestaltung dieses Verfahrens zur Erzeugung von dampfförmigen und für den anschließenden erfindungsgemäßen Einsatz bei der Gasphasenoxidation am Katalysator brauchbaren Molybdänverbindungen besteht darin, neben Isobuttersäure auch noch Sauerstoff, z.B. in Form von Luft, und gegebenenfalls weitere Intergase, wie auch Wasserdampf, mit über die z.B. MoO₃-haltige Schüttung zu leiten (s. dazu auch DE-C 25 50 979 = US-A-4 081 465). Dadurch wird eine Inaktivierung der Molybdänquelle, z.B. des MoO₃, und damit eine verminderte bzw. weithgehend zurückgehende Bildung flüchtiger Molybdänverbindungen vermieden. Die Inaktivierung der Molybdänquelle kann beispielsweise durch Verkokung z.B. der MoO₃-Oberflächen oder gegebenenfalls auch durch Reduktionsvorgänge verursacht werden.

Das Mol-Verhältnis von Isobuttersäure zu Sauerstoff im über die Molybdänquelle zu leitenden Gasgemisch, kann in weiten Grenzen variieren z.B. von 1 : 0,1 bis 1 : 2, vor allem 1 : 0,5 bis 1 : 1,5. Es wird insbesondere bestimmt von der Temperatur des z.B. MoO₃-haltigen Bettes, die im Bereich von etwa 150 bis etwa 350 Grad C liegt, und durch welches das Gasgemisch geleitet wird. Durch diese Größen kann dann eine Molybdänmenge in Form von flüchtigen Molybdänverbindungen erzeugt und kontinuierlich eingestellt werden, die erfindungsgemäß den Molybdänverlust am Katalysator im Oxidationsreaktor ausgleicht.

Eine mögliche Inaktivierung der Molybdänquelle, z.B. des MoO₃, für die Bildung flüchtiger Molybdänverbindungen beim Überleiten von Isobuttersäure bei 150 bis 350 Grad C, kann auch so verhindert werden, daß wechselweise Isobuttersäure, gegebenenfalls verdünnt mit inerten Zusätzen, und Sauerstoff, z.B. in Form von Luft, über die Quelle der zu bildenden flüchtigen Molybdänverbindungen bei Temperaturen von etwa 150 bis 350 Grad C geleitet werden.

Anstelle von Isobuttersäure lassen sich nach den vorstehend geschilderten Techniken auch mit anderen organischen, besonders OH-Gruppen-haltigen Verbindungen, wie beispielsweise Essigsäure oder Alkohlen wie Methanol oder Ethylenglykol, aber auch mit Aceton, aus einer Molybdänquelle, wie insbesondere MoO₃, flüchtige Molybdänverbindungen erzuegen, die dann erfindungsgemäß in die katalytische Oxidationsreaktion, z.B. die oxidative Dehydrierung von Isobuttersäure, eingeführt werden.

Es wurde gefunden, daß die Aufrechterhaltung der katalytischen Wirksamkeit der Heteropolysäure-Katalysatoren erreicht wird, wenn diesen während des Betreibens der katalytischen Reaktion Molybdän in Mengen von 0,003 bis 1 g pro kg der bei der Oxidationsreaktion an diesem Katalysator umzusetzenden organischen Substanz, wie z.B. der Isobuttersäure bei der oxidativen Dehydrierung zu Methacrylsäure, ständig oder gegebenenfalls auch zeitweilig in unregelmäßigen bzw. regelmäßigen Intervallen als Molybdänverbindungen zugeführt wird.

Insbesondere wird das Molybdän zur Katalysatorstabilisierung bzw. zur Katalysatorregenerierung in Mengen von 0,01 bis 0,5 g und vor allem in Mengen von 0,05 bis 0,15 g pro kg der an diesem Katalysator oxidativ umzusetzenden organischen Verbindung und in Form einer Molybdänverbindung, vorzugsweise in Form einer verdampfbaren organischen und/oder oxidischen Verbindung, in den Reaktor geführt.

Beispielhaft wird aus einer Schüttung von 24 g MoO₃, Partikelgröße 1 bis 3 mm, verteilt auf eine Schüttlänge von 15 cm, in Abhängigkeit von der Temperatur der MoO₃-Schüttung und dem Partialdruck der Isobuttersäure, nach Bestimmung in einer der MoO₃-Schüttung nachgeschalteten Waschvorrichtung, Molybdän in Mengen von 0,003 bis 1 g durch 1 000 g übergeleitete Isobuttersäure mitgenommen. Die Bestimmung des mitgenommenen Molybdäns erfolgt durch atomabsorptionsspektrometrische Bestimmung mit flammenloser Technik aus Lösung.

Auch die erfindungsgemäße Mitführung des Katalyssatorelements Phosphor bei der Oxidationsreaktion am Heteropolysäurekatalysator, vorzugsweise in Form einer Phosphorverbindung, beispielsweise als Phosphorsäure oder P₂O₅, insbesondere als organische Phosphorverbindung, vor allem eine solche

4

Phosphorverbindung mit Estergruppen zur Regenerierung - läßt sich mit bekannten Verbindungen und nach bekannten Methoden durchführen.

Als Phosphorverbindungen kommen hierzu solche der verschiedensten Oxidationsstufen des Phosphors, insbesondere die 3- oder 5-wertigen Verbindungen in Frage. Genannt Sien z.B. Trimethylphosphit (Kp 124 Grad C), Triaethylphosphat (Kp 215 Grad C) und vor allem Dimethylmethanphosphonat (Kp 154 Grad C). Die Verbindungen werden in dampfförmiger Form vorzugsweise kontinuierlich und mit weiteren Komponenten verdünnt in den katalysatorhaltigen Reaktor eingebracht, wobei sie in diesen destilliert oder auch sublimiert werden können.

Es wurde gefunden, daß die Aktivitätsstabilisierung und/oder die Reaktivierung der Heteropolysäure-Katalysatoren erreicht wird, wenn während des Betreibens der katalytischen Reaktion auf 1 000 Gewichtsteile der umzusetzenden organischen Verbindung, wie insbesondere der Isobuttersäure eine organische Phosphorverbindung, wie beispielsweise Triaethylphosphat oder insbesondere Dimethylmethanophosphonat, in Mengen (als Gewichtsteile) von 0,001 bis 1,0, vorzugsweise in Mengen von 0,005 bis 0,5 und besonders von 0,2 bis 0,5 mitgeführt werden. Höhere Mengen an mitgeführten Phosphorverbindungen führen zu einer deutlichen bis starken Desaktivierung des Katalysators.

Unter den bei der Oxidehydrierung und damit auch für die Reaktivierung herrschenden Bedingungen - 250 bis 400 Grad C, in Gegenwart von Sauerstoff und wie weiter schon in der DE-A-36 26 255 beschrieben, vorteilhaft auch die Gegenwart von Wasserdampf - werden die eingebrachten Phsophorverbindungen durch hydrolytisch-oxidative Reaktionen möglicherweise weiter umgesetzt und gegebenenfalls in für die Regenerierung der Heteropolysäure-Katalysatoren besonders brauchbare Phosphorverbindungen überführt.

Ist der Ausbau von gebrauchtem Katalysator aus dem Reaktor notwendig oder kann man laufend gebrauchten Katalysator aus einem entsprechend betriebenen Reaktor, beispielsweise einem Wirbelschichtreaktor, entnehmen, dann kann die Ergänzung fehlender Katalysatorbestandteile durch Zusatz entsprechender Verbindungen auch bei Temperaturen von 0 bis 100 Grad C, insbesondere bei Normaltemperatur, d.h. bei Temperaturen von etwa 0 bis 50 Grad C durchgeführt werden. Dies geschieht durch Vermischen des gebrauchten Katalysators mit den notwendigen Zusätzen, insbesondere in Form der oben genannten Verbindungen, gegebenenfalls in Gegenwart von Wasser zur besseren Verteilung der zu mischenden und letztlich zur Reaktion miteinander bestimmten Komponenten.

Der so aufgearbeitete, gebrauchte Katalysator wird dann in sauerstoffhaltiger, oxidierend wirkender Atmosphäre auf 200 bis 400 Grad C erhitzt, wobei sich der Katalysator praktisch wieder vollkommen und schnell regeneriert. Vorteilhaft, jedoch nicht zwingend, wird das Erhitzen auf 200 bis 400 Grad C in dem bei der anschließenden katalytischen Oxidation zu verwendenden Reaktorsystem ausgeführt. Ein ausgebauter bzw. ausgekreister und dann mit katalytisch notwendigen Elementen ergänzter Katalysator, wird vorteilhaft kontinuierlich in einen Reaktor mit bewegtem Bett eingekreist.

Das erfindungsgemäße Verfahren gestattet gegenüber dem Stand der Technik gebrauchten und desaktivierten Molybdo-Heteropolysäure-Katalysator wesentlich einfacher und schneller zu regenerieren und/oder diesen in der katalytischen Gasphasenoxidation vollwertig zu erhalten.

Das Verfahren der Erfindung ist bedeutsam für die oxidative Dehydrierung von Isobuttersäure oder ihrer Ester, zu Methacrylsäure oder ihrer Ester, die vorteilhaft an Katalysatoren auf der Basis von Heteropolysäuren des Molybdäns, auch von Salzen dieser Heteropolysäuren durchgeführt wird. Die Oxidehydrierung beispielsweise von Isobuttersäure, wird an diesen Katalysatoren im Temperaturbereich von etwa 250 bis 400 Grad C in Gegenwart von 1 bis 4 Mol Sauerstoff pro Mol Isobuttersäure durchgeführt, wobei weitere Inertgase wie u.a. Stickstoff oder Helium oder Wasserdampf oder $CO_2$ zugegen sein können. Die katalytische Umsetzung kann beispielsweise in Festbettreaktoren, wie Rohrreaktoren, oder in Reaktoren mit bewegtem Bett, wie Wirbelschichtreaktoren, durchgeführt werden. (Ullmanns Enzyklopädie der technischen Chemie, 4. Auflage, Verlag Chemie Weinheim New York, Band 13, Seiten 539 bis 542).

Vorzugsweise wird die katalytische Substanz in Gegenwart inerter Materialien, auf diese aufgebracht oder mit solchen verdünnt, in den Reaktoren angewendet (Ullmanns Enzykopädie der technischen Chemie, 4. Auflage, Verlag Chemie Weinheim New York, Band 13, Seiten 558 bis 565).

In den folgenden Beispielen wird die Erfindung an P-Mo-V-haltigen Heteropolysäure-Katalysatoren bei der Oxidehydrierung von Isobuttersäure zu Methacrylsäure gezeigt.

Allgemeine Versuchsdurchführung der Oxidehydrierung bei den Beispielen 1 bis 4 und den zugehörigen Vergleichsbeispielen.

Ein dampfförmiges Gemisch aus Isobuttersäure, Sauerstoff und Stickstoff im Molverhältnis 1 : 1,5 : 7,7 wird in einem Kreislaufreaktor nach DE-A-30 19 731 bei 340 Grad C und einer Verweilzeit von 1 sec an einem Katalysator zur Reaktion gebracht. Die Belastung des Katalysators beträgt dabei jeweils 1 250 g Isobuttersäure pro 1 000 g katalytischer Masse und Stunde. Das Reaktionsgas wird laufend gaschromatographisch analysiert und aus diesen Werten Isobuttersäure-Umsatz und Methacrylsäure-Selektivität ermittelt.

Vergleichsbeispiel 1

Ein $Cu_{0,15}H_{3,7}PMo_{11}VO_{40}$-Katalysator, hergestellt gemäß EP-B 113 084, verdünnt mit 30 Gew.-% Kieselsäure (Kieselgur: Aeroil $^R$ = 5 : 1 Gew.-Teile), zeigt bei der Isobuttersäure-Oxidehydrierung und bei einer Methacrylsäure-Selektivität von 74% in den ersten 25 Stunden einen Isobuttersäure-Umsatz von 82 %, der dann ständig abfällt und nach 96 Reaktionsstunden einen Wert von 76,5 % erreicht.

Beispiel 1

Ein Gemisch aus 141,3 g eines gebrauchten $Cu_{0,15}H_{3,7}PMo_{11}$-$V_1O_{40}$-Katalysators und 12,1 g $MoO_3$ - dies entspricht 38 % der verlorengegangenen katalytischen Materials - wird in 1 000 g $H_2O$ unter Rühren 2 Stunden zum Sieden erhitzt. Danach wird die tiefblaue Suspension bis zur Paste eingeengt. Nach 1 Stunde bei 110 Grad C und 3 Stunden bei 200 Grad C im Trockenschrank erfolgt das 3-stündige Erhitzen des Katalysators im Reaktorofen bei 300 Grad C in Gegenwart von Luft. Der Katalysator liegt nun in olivgrüner Farbe vor.

Unter den oben genannten Reaktionsbedingungen zeigt der regenerierte Katalysator folgende Oxidehydrierungsergebnisse:

| Versuchszeit (h) | Umsatz (%) |
|---|---|
| 25 | 82,2 |
| 48 | 81,1 |
| 82 | 79,9 |
| 109 | 79,1 |

Die Methacrylsäure-Selektivität beträgt 73 %.

Vergleichsbeispiel 2

Ein $H_5PMo_{10}V_2O_{40}$-Katalysator, der mit 30 % Kieselsäure (Kieselgur : Aerosil $^R$ = 5 : 1) verdünnt ist, hergestellt nach DE-A-27 22 375, wird unter den angegebenen Oxidehydrierungsbedingungen geprüft.

| Versuchszeit (h) | Umsatz (%) |
|---|---|
| 6 | 81,4 |
| 23 | 79,2 |
| 49 | 79,0 |
| 72 | 77,1 |

Die Methacrylsäure-Selektivität liegt bei 72 %.

Beispiel 2

Ein Gemisch aus 164,2 g eines gebrauchten $H_5PMo_{10}V_2O_{40}$-Katalysators und 11,2 g $MoO_3$ - dies entspricht 100 % des verlorengegangenen Materials - wird in 1 578 g $H_2O$ unter Rühren 6 Stunden zum Sieden erhitzt. Nach Zugabe von 5 % Aerosil $^R$ wird das tiefblaue Gemisch bis zur Paste eingeengt. Anschließend wird die Präparation 1 Stunde bei 110 Grad C und 3 Stunden bei 200 Grad C im Trockenschrank erhitzt. Die Oxidation erfolgt bei 300 Grad C im Reaktorofen (3 Stunden) unter Durchleiten von Luft.

Unter den Reaktionsbedingungen des Vergleichskatalysators 2 zeigt der regenerierte Katalysator folgende Ergebnisse:

| Versuchszeit (h) | Umsatz (%) |
|---|---|
| 7 | 80,6 |
| 27 | 79,8 |
| 72 | 77,5 |

Die Methacrylsäure-Selektivität beträgt 72 %.

Vergleichsbeispiel 3

Ein $Cu_{0,2}H_{4,6}PMo_{10}V_2O_{40}$-Katalysator, der mit 30% Kieselsäure (Kieselgur : Aerosil [R] = 5 : 1) verdünnt ist, wird unter den oben genannten Oxidehydrierungsbedingungen getestet. Ergebnise:

| Versuchszeit (h) | Umsatz (%) |
|---|---|
| 7 | 78;2 |
| 26 | 72,9 |
| 49 | 69,1 |
| 78 | 64,7 |

Die Methacrylsäure-Selektivität beträgt 75 %.

Beispiel 3

Ein Gemisch von 166,2 eines gebrauchten $Cu_{0,2}H_{4,6}PMo_{10}$-$V_2O_{40}$-Katalysators und 6,7 g $MoO_3$ - dies entspricht 90 % des verlorengegangenen katalytischen Materials - wird in 1 550 g $H_2O$ unter Rühren 6 Stunden zum Sieden erhitzt.

Danach werden 5 Gew.-% Aerosil [R] zugegeben und das Gemisch zur Paste eingeengt. Nach 1 Stunde bei 110 Grad C und 3 Stunden bei 200 Grad C im Trockenschrank wird der Katalysator in Gegenwart von Luft 3 Stunden im Muffelofen bei 310 Grad C oxidiert. Nach der Aufarbeitung liegt der Katalysator in olivgrüner Farbe vor. Unter den Reaktionsbedingungen des Vergleichskatalysators ergibt der regenerierte Katalysator folgende Resultate:

| Versuchszeit (h) | Umsatz (%) |
|---|---|
| 6 | 77,5 |
| 27 | 76,3 |
| 80 | 75,5 |

Die Methacrylsäure-Selektivität liegt bei 73 %.

Vergleichsbeispiel 4

Ein $Cu_{0,1}Cs_1H_{2,8}PMo_{11}V_1O_{40}$-Katalysator, der mit 30 % Kieselsäure (Kieselgur : Aerosil [R] = 5 : 1) verdünnt ist, wird unter oben angegebenen Oxidehydrierungsbedingungen getestet. Ergebnisse:

| Versuchszeit (h) | Umsatz (%) |
|---|---|
| 6 | 82,2 |
| 25 | 79,8 |
| 73 | 76,4 |

Die Methacrylsäure-Selektivität beträgt 75 %.

Beispiel 4

Ein Gemisch von 90,3 g eines gebrauchten $Cu_{1,0}Cs_1H_{2,8}$-$PMo_{11}V_1O_{40}$-Katalysators und 11,9 g $MoO_3$ - dies entspricht 90 % des verlorengegangenen katalytischen Materials - wird in 920 g $H_2O$ unter Rühren 1 Stunde zum Sieden erhitzt.

Anschließend wird das Gemisch zur Paste eingeengt. Nach 1 Stunde bei 110 Grad C und 3 Stunden bei 200 Grad C im Trockenschrank wird der Katalysator 3 Stunden in Gegenwart von Luft im Muffelofen bei 280 Grad C oxidiert. Nach der Aufarbeitung liegt der Katalysator in olivgrüner Farbe vor.

Unter den Reaktionsbedingungen des Vergleichskatalysators ergibt der regenerierte Katalysator folgende Resultate:

| Versuchszeit (h) | Umsatz (%) |
|---|---|
| 20 | 82,3 |
| 43 | 81,8 |
| 67 | 81,4 |

Die Methacrylsäure-Selektivität liegt bei 74 %.

Beispiel 5

a) Vergleich

Werden bei 316 Grad C und einem Druck von 1 bar über 2 g $H_6V_3Mo_9PO_{40}$ mit einer Partikelgröße von 400 bis 600 µm, verdünnt mit Glaskugeln derselben Partikelgröße im Gewichtsverhältnis 1 : 4, und auf einer Schüttlänge von 10 cm angeordnet, pro Stunde 1,5 Mol eines Gasgemisches bestehend aus 0,06 Mol Stickstoff, 1,155 Mol Helium, 0,045 Mol Isobuttersäure und 0,24 Mol Sauerstoff geleitet, so werden nach gaschromatographischen Analysen anfänglich 88 % der eingeleiteten Isobuttersäure umgesetzt. Dabei entsteht Methacrylsäure in einer Ausbeute von 60 % d.Th., d.h. mit einer Selektivität von 68 %. Die Aktivität des Katalysators fällt im Laufe von 20 Stunden Reaktionsdauer stetig ab, wonach bei gleichbleibender Selektivität, der Isobuttersäureumsatz auf 66 % gesunken ist.

b) Erfindungsgemäßes Vorgehen

Wird nun das unter a) angegebene Gasgemisch, jedoch ohne den Sauerstoffzusatz, zunächst über 24 g eines auf 250 Grad C gehaltenen $MoO_3$ geleitet und nach Verlassen der $MoO_3$-Zone ("Mo-Sättiger") mit der unter a) angegebenen Sauerstoffmenge gemischt und dieses so hergestellte Gasgemisch wie im Vergleich a) angegeben, umgesetzt, so wird innerhalb einer Reaktionsdauer von 25 Stunden kein Aktivitätsabfall festgestellt. Bei einem Isobuttersäureumsatz von 88% wird Methacrylsäure in einer Ausbeute von 61% d.Th. bei einer gleichbleibenden Selektivität von 69% erhalten.

Beispiel 6

Über 2 g $H_5V_2Mo_{10}PO_{40}$, angeordnet wie der homologe Heteropolysäurekatalysator aus Beispiel 5, werden bei 309 Grad C pro Stunde 1,5 Mol eines Gasgemisches geleitet, das hergestellt ist aus

a) 0,06 Mol Stickstoff, 1,125 Mol Helium un 0,075 Mol Isobuttersäure, beladen mit Molybdän nach gleichem Vorgehen wie in Beispiel 5, b) beschrieben, und
b) 0,24 Mol Sauerstoff.

Während einer Reaktionsdauer von 60 Stunden wird durch gaschromatographische Analysen ein konstanter Isobuttersäureumsatz von 84 % und eine konstante Methacrylsäureausbeute von 53 % d.Th., was einer Selektivität von 63 % entspricht, gemessen.

Ohne Mitführung von Molybdän aus dem $MoO_3$-Reaktor ("Mo-Sättiger") fällt die Aktivität des Katalysators schon innerhalb der ersten 10 Reaktionsstunden stark ab und erreicht dabei einen Wert des Isobuttersäureumsatzes von 66 %.

Beispiel 7

Ein wie in den Beispielen 5 und 6 beschrieben angeordneter $H_5V_2Mo_{10}PO_{40}$-Katalysator erreicht nach einer Reaktionsdauer von 220 Stunden bei der Oxidehydrierung von Isobuttersäure zu Methacrylsäure eine Methacrylsäureausbeute von noch 48% d.Th.. Diese fällt innerhalb von weiteren 30 Reaktionsstunden auf 24 % d.Th. ab. Jetzt wird der sauerstoffreie Teil des im Beispiel 6 beschriebenen Gasgemisches in den wie in den Beispielen 5 und 6 beschriebenen, auf 320 Grad C gehaltenen, $MoO_3$-Reaktor geleitet, und dann das diesen Reaktor verlassende "Mo"-haltige Gasgemisch mit der in Beispiel 6 angegebenen Sauerstoffmenge versetzt. Gaschromatographisch läßt sich feststellen, daß innerhalb weiterer 20 Reaktionsstunden keine weitere Desaktivierung des Katalysators stattfindet.

Beispiel 8

Über 59 kg eines Heteropolysäure-Katalysators aus 12 Gew.-% $H_{3,6}Cu_{0,2}Mo_{11}PVO_{40}$ und 88 Gew.-% $Al_2O_3$-Träger, werden bei einer Katalysatortemperatur von 350 Grad C pro Stunde 18300 1 eines Gasgemisches bestehend aus Isobuttersäure, Sauerstoff, Stickstoff und Wasser im Molverhältnis 1/1,5/5,64/1 geleitet.

Innerhalb einer Betriebszeit von 550 Stunden ist ein stetiger Abfall des Isobuttersäureumsatzes von anfänglich 95 % auf 73 % durch gaschromatographische Analysen feststellbar. Innerhalb dieser Betriebszeit fällt die Methacrylsäureselektivität von anfänglich 70 % auf 65 % ab.

Zu diesem Zeitpunkt werden dem Gasgemisch in der ersten Stunde 4 g Dimethylmethanposphonat zudosiert und anschließend wird über einen Zeitraum von 500 Stunden ein Gasgemisch über den Katalysator geleitet, das abweichend von den obigen Angaben mit 0,01 Gew.-% Dimethylmethanphosphonat-haltiger Isobuttersäure hergestellt ist. Durch die Maßnahmen steigt der Umsatz an Isobuttersäure innerhalb weniger Stunden auf 80 % an und bleibt, wie die Methacrylsäureselektivität mit 65%, während der gesamten Betriebsdauer konstant.

Wird die Zugabe der Phosphorverbindung unterlassen, fällt der Isobuttersäureumsatz innerhalb der etwa 1 000 Stunden Reaktionsdauer auf Werte um 60 % und die Methacrylsäureselektivität auf Werte um 50 % ab.

Beispiel 9

In einem weiteren Versuch in einem Laborreaktor mit 2 kg ges gleichen Katalysators wie in Beispiel 8 beschrieben, bei einer Reaktionstemperatur von 360 Grad C und dessen Belastung mit einem Gasgemisch von 620 l/Stunde, bestehend aus Isobuttersäure, Sauerstoff, Stickstoff und Wasser im Molverhältnis 1/1,5/5,64/1, werden innerhalb von 1465 Stunden Betriebsdauer ein Umsatzrückgang der Isobuttersäure um 7 % und ein Selektivitätsrückgang der Methacrylsäure auf 52 %, d.h. um 18 %, durch gaschromatographische Analysen festgestellt.

Jetzt werden innerhalb vom 46 Stunden mit dem Gasgemisch 1,74 g Dimethylmethanphosphonat über den Katalysator geführt. Dabei wird die Ausgangsaktivität mit einem Isobuttersäureumsatz von 90 % wieder

EP 0 284 872 B1

erreicht und die Methacrylsäureselektivität steigt auf 66 % an.

Beispiel 10

Nach der gleichen Versuchsdurchführung wie in Beispiel 9 beschrieben wird nach einer Reaktionsdauer von 1006 Stunden, nachdem der Isobuttersäureumsatz von 90 % auf 60 % und die Methacrylsäureselektivität von 70 % auf 43,3 % abgesunken sind, dem Gasgemisch Dimethylmethanposphonat in einer Menge von 0,1 Gew.-% bezogen auf die Isobuttersäure zugegeben. Nach einer anfänglich kurzen Aktivitäts- und Selektivitätsregenerierung, wird mit fortschreitender Dosierung eine vollkommene Desaktivierung und Deselektivierung des Katalysators erreicht. Dies zeigt, daß die hier dosierte Dimethylmethanphosphonat-Menge zu hoch bemessen ist.

**Patentansprüche**

1. Verfahren zur Aktivitätsstabilisierung und/oder zur Regenerierung von Heteropolysäure-Katalysatoren, die Molybdän, Phosphor und Vanadium als wesentliche Elemente, sowie gegebenenfalls weitere metallische Elemente als Kationen enthalten, bei ihrer Verwendung als Oxidationskatalysatoren in Gasphasenreaktionen,

    dadurch gekennzeichnet,

    daß dem Katalysator Verbindungen von Katalysatorbestandteilen zugeführt werden und dieser in sauerstoffhaltiger, oxidierend wirkender Atmosphäre auf 200 bis 400 Grad C erhitzt wird.

2. Verfahren nach Anspruch 1, dadurch gekennzeichnet, daß die katalytische Gasphasenoxidation ein Verfahren zur Herstellung von Methacrylsäure oder ihrer niederen Ester durch oxidative Dehydrierung von Isobuttersäure oder ihrer niederen Ester ist und bei 250 bis 400 Grad C durchgeführt wird.

3. Verfahren nach den Ansprüchen 1 und 2, dadurch gekennzeichnet, daß die Zuführung von Verbindungen von Katalysatorbestandteilen währen der katalytischen Gasphasenoxidation, wie der oxidativen Dehydrierung von Isobuttersäure oder ihrer niederen Ester zu Methacrylsäure oder ihrer niederen Ester, erfolgt.

4. Verfahren nach der Ansprüchen 1 bis 3, dadurch gekennzeichnet, daß mit den umzusetzenden Komponenten Verbindungen von Heteropolysäureelementen, gasförmig, bei 250 bis 400 Grad C über den aus den Heteropolysäureverbindungen gebildeten Katalysator geleitet werden.

5. Verfahren nach den Ansprüchen 1 bis 4, dadurch gekennzeichnet, daß Molybdän- und/oder Phosphorverbindungen, gasförmig, bei 250 bis 400 Grad C mit den umzusetzenden Komponenten über den Katalysator geleitet werden.

6. Verfahren nach den Ansprüchen 1 bis 5, dadurch gekennzeichnet, daß Molybdän als gasförmige Molybdänverbindungen in einer Menge von 0,003 bis 1 g, insbesondere in einer Menge von 0,01 bis 0,5 g und vor allem in einer Menge von 0,05 bis 0,15 g pro 1,000 g der umzusetzenden organischen Verbindung mitgeführt wird.

7. Verfahren nach den Ansprüchen 1 bis 6, dadurch gekennzeichnet, daß die gasförmigen Molybdänverbindungen durch Überleiten von Isobuttersäure über Molybdänoxide, besonders über $MoO_3$, bei Temperaturen von 100 bis 350 Grad C hergestellt werden.

8. Verfahren nach den Ansprüchen 1 bis 7, dadurch gekennzeichnet, daß die gasförmigen Molybdänverbindungen durch Überleiten eines Gasgemischs, das Isobuttersäure und Sauerstoff enthält, über Molybdänoxide, besonders über $MOO_3$, bei Temperaturen von 100 bis 350 Grad C hergestelllt werden.

9. Verfahren nach den Ansprüchen 1 bis 7, dadurch gekennzeichnet, daß bei der Herstellung der gasförmigen MOlybdänverbindungen wechselweise Isobuttersäure-haltige Gasgemische und Sauerstoffhaltige Gasgemische über das Molybdänoxid-Bett bei 100 bis 350 Grad C geleitet werden.

10

**10.** Verfahren nach den Ansprüchen 1 bis 9, dadurch gekennzeichnet, daß zur Herstellung gasförmiger Molybdänverbindungen anstelle von Isobuttersäure andere organische Verbindungen, insbesondere solche mit OH-Gruppen, wie Essigsäure oder Methanol oder Ethylenglykol oder auch Aceton, eingesetzt werden.

**11.** Verfahren nach den Ansprüchen 1 bis 5, dadurch gekennzeichnet, daß als gasförmige Phosphorverbindung eine organische Phosphorverbindung mit Estergruppen mitgeführt wird.

**12.** Verfahren nach den Ansprüchen 1 bis 5 und 11 , dadurch gekennzeichnet, daß als organische Phosphorverbindung Dimethylmethanphosphonat mitgeführt wird.

**13.** Verfahren nach den Ansprüchen 1 bis 5 sowie 11 und 12 dadurch gekennzeichnet, daß die organische Phosphorverbindung in Mengen von 0,001 bis 1,0 g, vorzugsweise in Mengen von 0,005 bis 0,5 g und besonders in Mengen von 0,02 bis 0,5 g pro 1 000 g der umzusetzenden organischen Verbindung mitgeführt wird.

**14.** Verfahren nach den Ansprüchen 1 und 2, dadurch gekennzeichnet, daß die Zuführung von Verbindungen von Katalysatorbestandteilen zur Ergänzung von Katalysatorelementen-Verlusten und das Erhitzen in sauerstoffhaltiger, oxidierend wirkender Atmosphäre räumlich getrennt voneinander durchgeführt werden.

**15.** Verfahren nach den Ansprüchen 1, 2 und 14, dadurch gekennzeichnet, daß zur Ergänzung von Katalysatorelementen-Verlusten oxidische Verbindungen von Molybdän und/oder Phosphor und gegebenenfalls von Vanadium eingesetzt werden.

## Claims

**1.** Process for stabilising the activity of and/or for regenerating heteropolyacid catalysts which contain molybdenum, phosphorus and vanadium as essential elements, possibly as well as other metallic elements as cations, when they are used as oxidation catalysts in gas phase reactions, characterised in that compounds of catalyst components are fed into the catalyst and the latter is heated in an oxygen-containing atmosphere with an oxidising effect at 200 to 400° C.

**2.** Process as claimed in claim 1, characterised in that the catalytic gas phase oxidation is a process for preparing methacrylic acid or the lower esters thereof by oxidative dehydrogenation of isobutyric acid or the lower esters thereof and is carried out at 250 to 400° C.

**3.** Process as claimed in claims 1 and 2, characterised in that the compounds of catalyst components are fed in during the catalytic gas phase oxidation, such as the oxidative dehydrogenation of isobutyric acid or the lower esters thereof to obtain methacrylic acid or the lower esters thereof.

**4.** Process as claimed in claims 1 to 3, characterised in that together with the components which are to be reacted, compounds of heteropolyacid elements in gaseous form are passed at 250 to 400° C over the catalyst which is formed from the heteropolyacid compounds.

**5.** Process as claimed in claims 1 to 4, characterised in that molybdenum and/or phosphorus compounds in gaseous form are passed at 250 to 400° C over the catalyst together with the components to be reacted.

**6.** Process as claimed in claims 1 to 5, characterised in that molybdenum is carried along in the form of gaseous molybdenum compounds in a quantity of from 0.003 to 1 g, more particularly from 0.01 to 0.5 g and especially in a quantity of from 0.05 to 0.15 g per 1000 g of the organic compound which is to be reacted.

**7.** Process as claimed in claims 1 to 6, characterised in that the gaseous molybdenum compounds are prepared by passing isobutyric acid over molybdenum oxides, more particularly over $MoO_3$, at temperatures of from 100 to 350° C.

8. Process as claimed in claims 1 to 7, characterised in that the gaseous molybdenum compounds are prepared by passing a gas mixture which contains isobutyric acid and oxygen over molybdenum oxides, particularly $MoO_3$, at temperatures of from 100 to 350°C.

9. Process as claimed in claims 1 to 7, characterised in that, in the preparation of the gaseous molybdenum compounds, gas mixtures containing isobutyric acid and gas mixtures containing oxygen are alternately passed over the molybdenum oxide bed at 100 to 350°C.

10. Process as claimed in claims 1 to 9, characterised in that in order to prepare gaseous molybdenum compounds, instead of isobutyric acid other organic compounds are used, particularly those with OH groups such as acetic acid or methanol or ethylene glycol or acetone.

11. Process as claimed in claims 1 to 5, characterised in that an organic phosphorus compound with ester groups is included as the gaseous phosphorus compound.

12. Process as claimed in claims 1 to 5 and 11, characterised in that dimethylmethanephosphonate is introduced as an organic phosphorus compound.

13. Process as claimed in claims 1 to 5 and 11 and 12, characterised in that the organic phosphorus compound is used in quantities of from 0.001 to 1.0 g, preferably in quantities of 0.005 to 0.5 g and more particularly in quantities of from 0.02 to 0.5 g per 1000 g of the organic compound which is to be reacted.

14. Process as claimed in claims 1 and 2, characterised in that the introduction of compounds of catalyst components to supplement losses of catalyst elements and the heating in an oxygen-containing atmosphere with an oxidising effect are carried out spatially separate from each other.

15. Process as claimed in claims 1, 2 and 14, characterised in that in order to supplement losses of catalyst elements oxidic compounds of molybdenum and/or phosphorus and optionally vanadium are used.

## Revendications

1. Procédé de stabilisation de l'activité et/ou de régénération de catalyseurs, qui sont des hétéropolyacides contenant comme cations du molybdène, du phosphore et du vanadium comme éléments essentiels ainsi qu'éventuellement d'autres éléments métalliques, lorsqu' on les utilise comme catalyseurs d'oxydation dans des réactions en phase gazeuse, caractérisé en ce qu'on alimente le catalyseur en composés des constituants du catalyseur et en ce qu'on chauffe ce catalyseur à une température de 200 à 400°C dans une atmosphère oxydante contenant de l'oxygène.

2. Procédé suivant la revendication 1, caractérisé en ce que l'oxydation catalytique en phase gazeuse est un procédé de fabrication de l'acide méthacrylique ou de ses esters inférieurs par déshydrogénation par oxydation de l'acide isobutyrique ou de ses esters inférieurs et en ce qu'elle est réalisée à une température de 250 à 400°C.

3. Procédé suivant la revendication 1 ou 2, caractérisé en ce que l'alimentation en composés des constituants du catalyseur a lieu pendant l'oxydation catalytique en phase gazeuse, telle que la déshydrogénation par oxydation de l'acide isobutyrique ou de ses esters inférieurs en acide méthacrylique ou en les esters inférieurs de celui-ci.

4. Procédé suivant l'une des revendications 1 à 3, caractérisé en ce que des composés des éléments de l'hétéropolyacide sont amenés sous forme gazeuse et à une température de 250 à 400°C sur le catalyseur formé des composés de l'hétéropolyacide, conjointement avec les composants à faire réagir.

5. Procédé suivant l'une des revendications 1 à 4, caractérisé en ce que des composés du molybdène et/ou du phosphore sont amenés sous forme gazeuse et à une température de 250 à 400°C sur le catalyseur, conjointement avec les composants à faire réagir.

6. Procédé suivant l'une des revendications 1 à 5, caractérisé en ce que, pour 1000 g du composé organique à faire réagir, on amène simultanément du molybdène sous forme de composés du molybdène à l'état gazeux, en une quantité de 0,003 à 1 g, en particulier en une quantité de 0,01 à 0,5 g et surtout en une quantité de 0,05 à 0,15 g.

7. Procédé suivant l'une des revendications 1 à 6, caractérisé en ce que l'on prépare les composés du molybdène à l'état gazeux en faisant passer de l'acide isobutyrique sur des oxydes de molybdène, en particulier sur $MoO_3$, à des températures de 100 à 350° C.

8. Procédé suivant l'une des revendications 1 à 7, caractérisé en ce que l'on prépare les composés du molybdène à l'état gazeux en faisant passer un mélange gazeux contenant de l'acide isobutyrique et de l'oxygène sur des oxydes de molybdène, en particulier sur $MoO_3$, à des températures de 100 à 350° C.

9. Procédé suivant l'une des revendications 1 à 7, caractérisé en ce que, pour préparer les composés du molybdène à l'état gazeux, on fait passer alternativement des mélanges gazeux contenant de l'acide isobutyrique et des mélanges gazeux contenant de l'oxygène sur le lit d'oxydes de molybdène à une température de 100 à 350° C.

10. Procédé suivant l'une des revendications 1 à 9, caractérisé en ce que, pour préparer les composés du molybdène à l'état gazeux, on utilise à la place de l'acide isobutyrique d'autres composés organiques, en particulier des composés organiques contenant des groupes OH, comme l'acide acétique ou le méthanol ou l'éthylèneglycol, ou encore l'acétone.

11. Procédé suivant l'une des revendications 1 à 5, caractérisé en ce que, comme composé du phosphore à l'état gazeux, on fait passer conjointement sur le catalyseur un composé organique du phosphore comprenant des groupes ester.

12. Procédé suivant l'une des revendications 1 à 5 et 11, caractérisé en ce que, comme composé organique du phosphore, on fait passer conjointement sur le catalyseur du méthanephosphonate de diméthyle.

13. Procédé suivant l'une des revendications 1 à 5 et 11 et 12, caractérisé en ce que, pour 1 000 g du composé organique à faire réagir, on amène conjointement le composé organique du phosphore en des quantités de 0,001 à 1,0 g, de préférence en des quantités de 0,005 à 0,5 g et notamment en des quantités de 0,02 à 0,5 g.

14. Procédé suivant la revendication 1 ou 2, caractérisé en ce que l'alimentation en composés des constituants du catalyseur pour compenser les pertes en éléments du catalyseur et le chauffage en atmosphère oxydante contenant de l'oxygène sont effectués séparément l'une de l'autre dans l'espace.

15. Procédé suivant l'une des revendications 1, 2 et 14, caractérisé en ce que, pour compenser les pertes en éléments du catalyseur, on introduit des composés oxydés du molybdène et/ou du phosphore et éventuellement du vanadium.